# EUROPEAN PATENT APPLICATION

(11) **EP 2 193 770 A1**
(43) Date of publication of application: **09.06.2010**
(21) Application number: 08778070.6
(22) Date of filing: 11.07.2008
(51) Int. Cl.: A61F 13/15, A61F 13/49, A61F 13/494, A61F 13/511

(54) **ABSORPTIVE ARTICLE**

(30) Priority: 28.09.2007 JP 2007255990
(71) Applicant: Unicharm Corporation, Ehime 799-0111 (JP)
(72) Inventor: MINATO, Hironao, Kanonji-shi Kagawa 769-1602 (JP); NAKAJIMA, Kaiyo, Kanonji-shi Kagawa 769-1602 (JP)
(74) Representative: Stark, Gordon Drummond
(86) International application number: PCT/JP2008/062554
(87) International publication number: WO 2009/041143

(57) **Abstract**

The present invention aims to provide an improved absorbent article wherein the area of a barrier sheet which comes into contact with the article wearer's skin is reduced, without the resulting features causing troubles, such as accidental rupture of the barrier sheet in the course of making the diaper. A diaper 1 as a typical example of the absorbent article comprises an absorbent chassis which, in turn, includes a cover sheet 6 and a liquid-absorbent structure 7, and a barrier sheet 8 allowing passage of body waste. The barrier sheet 8 is spaced from the liquid-absorbent structure 7 in the thickness direction under an effect of barrier sheet constricting means 33. The barrier sheet 8 is formed with front and rear passage openings 27, 28 defined by a middle portion 26 and the middle portion 26 is provided, in addition to the barrier sheet constricting means 33, with middle portion elastic members 38, 39 which function to constrict the middle portion 26 in a longitudinal direction Y.

## Description

### TECHNICAL FIELD

The present invention relates to an absorbent article and particularly to an absorbent article such as disposable diaper, toilet training pants or incontinent briefs.

### RELATED ART

Disposable diapers which have a specific construction adapted to protect the diaper wearer' s skin from contamination with body waste are well known, for example, from PCT International Application Publication No. 1997-510385 (JP 1997-510385W).
PATENT DOCUMENT 1: PCT International Application Publication No. 1997-510385 (JP 1997-510385W)

### DISCLOSURE OF THE INVENTION

### PROBLEM TO BE SOLVED BY THE INVENTION

JP 1997-510385W discloses a pant-type disposable diaper. This diaper comprises a pant-shaped cover sheet and a liquid-absorbent structure provided on the inner side of the cover-sheet, wherein the liquid-absorbent structure is provided thereon with a pair of sheet-like flaps spaced from each other in a transverse direction. The paired flaps are joined to each other at a joining portion which cooperates with the respective flaps to define a front passage opening and a rear passage opening. The flaps are provided along these front and rear passage openings with elastic members which are attached under tension to the respective flaps so as to extend in a longitudinal direction. Under contraction of these elastic members, the regions of the flaps defined between the elastic members and the opposite side edges of the flaps are spaced upward together with the join portion from the liquid-absorbent structure so that a pocket to receive urine is formed under the front passage opening and a pocket to receive faces is formed under the rear passage opening.

In the case of the diaper disclosed in JP 1997-510385W, at least the joining portion comes into contact with the wearer's skin. In order to alleviate a feeling of discomfort and undesirable irritation of the wearer's skin both due to contact of the joining portion with the wearer's skin, the area of the joining portion may be reduced. To reduce this area, it is conceivable to enlarge the front passage opening rearward and to enlarge the rear passage opening forward so that the dimensions of the joining portion in the longitudinal direction may be reduced. However, reduction of the longitudinal dimension of the joint portion will necessarily weaken the tensile strength of the join portion and, in consequence, there has been the problem that the join portion can be broken off during the step of cutting out the front and rear passage openings, for example, during press-processing in a process for making the diaper.

In view of the problem as has been described above, the present invention provides an improved absorbent article wherein the area of the portion coming in contact with the article wearer's skin may be reduced without inducing accidental rupture of the barrier sheet in the course of making the same.

### MEASURE TO SOLVE THE PROBLEM

According to one aspect of the present invention, there is provided an improved absorbent article comprising a liquid-absorbent chassis having a longitudinal direction, a transverse direction, a side facing the article wearer's body, a side facing the wearer' s garment, a front waist region, a rear waist region, a crotch region extending between said front and rear waist region and a liquid-absorbent structure provided in the crotch region, and a barrier sheet provided on the side facing the article wearer's body of the liquid-absorbent chassis which is adapted to be spaced from the liquid-absorbent chassis wherein the barrier sheet includes barrier sheet constricting means biasing the barrier sheet to be constricted in the longitudinal direction and a space which is formed between the barrier sheet and the liquid-absorbent structure as the liquid-absorbent structure is bowed in the longitudinal direction.

The improvement according to the this aspect of the present invention is **characterized in that** the barrier sheet comprises a pair of lateral portions opposed to and spaced from each other in the transverse direction, a middle portion connecting the lateral portions to each other, front and rear passage openings allowing passage of body waste into the space and, in addition to the barrier sheet constricting means, middle portion constricting means adapted to constrict the middle portion in the longitudinal direction.

According to one preferred embodiment, the middle portion constricting means comprises middle portion elastic members biasing the middle portion to be constricted in the longitudinal direction and wherein the middle portion elastic members extend in parallel to a longitudinal center line bisecting a dimension of the middle portion in the transverse direction and bonded under tension to the middle portion so as to extend across the middle portion in the longitudinal direction.

According to another preferred embodiment, the barrier sheet constricting means comprises barrier sheet elastic members extending in the longitudinal direction provided along respective inner edges defining the front and rear passage openings at least in the lateral portions.

According to a further preferred embodiment, the barrier sheet elastic members are provided so as to be convexly curved, in the middle portion, towards a longitudinal center line bisecting the dimension of the barrier sheet and to be gradually spaced from the inner edges as the elastic members get near to the longitudinal center line.

According to a further preferred embodiment, the middle portion constricting means is set to a stretch ratio higher than that of the barrier sheet elastic members.

The invention further provides an improvement **characterized in that** the barrier sheet comprises a pair of lateral portions opposed to and spaced from each other in the transverse direction, a middle portion connecting the lateral portions to each other, and front and rear passage openings defined in front and rear zones of the crotch region by the intermediary of the middle portion and allowing passage of body waste into the space, and barrier sheet constricting means comprising barrier sheet elastic members extending in the longitudinal direction provided along respective inner edges defining the front and rear passage openings in the lateral portions and provided close to a longitudinal center line bisecting the dimension of the barrier sheet, extending in parallel to the longitudinal center line in the middle portion.

### EFFECT OF THE INVENTION

According to the present invention, the barrier sheet is provided in the middle portion with the middle portion constricting means serving to constrict the middle portion in the longitudinal direction so that the area of the middle portion which actually comes into contact with the wearer' s skin can be effectively reduced and any feeling of discomfort, as well as any skin irritation due to such contact, may be effectively alleviated. Reduction of the skin contacting area is achieved, according to the invention, by constricting the longitudinal dimension of the middle portion under the effect of the constricting means instead of preliminarily reducing the longitudinal dimension of the middle portion in the course of forming the front and rear passage openings. Therefore, the tensile strength of the middle portion set in the step of forming the passage openings is sufficiently maintained to prevent the middle portion from being accidentally broken off.

### BRIEF DESCRIPTION OF THE DRAWINGS

[FIG. 1] Fig. 1 is a perspective view of a first embodiment of the present invention.
[FIG. 2] Fig. 2 is a sectional view taken along the line II-II in Fig. 1.
[FIG. 3] Fig. 3 is a plan view showing the diaper of Fig. 1 provided in a flat opened out arrangement.
[FIG. 4] Fig. 4 is an exploded perspective view of the plan view of the flat diaper of Fig. 3.
[FIG. 5] Fig. 5 is a scale-enlarged view of the barrier sheet according to a first embodiment.
[FIG. 6] Fig. 6 is a scale-enlarged view of the middle portion of the barrier sheet after contraction as viewed in a direction of the arrow VI in Fig. 2.
[FIG. 7] Fig. 7 is a scale-enlarged view of the barrier sheet according to a second embodiment.
[FIG. 8] Fig. 8 is a scale-enlarged view of the barrier sheet according to a third embodiment.

### IDENTIFICATION OF REFERENCE NUMERALS USED IN THE DRAWINGS

- 1: diaper
- 2: liquid-absorbent chassis
- 3: front waist region
- 4: rear waist region
- 5: crotch region
- 6: cover sheet
- 7: liquid-absorbent structure
- 8: barrier sheet
- 26: middle portion
- 27: front passage opening
- 28: rear passage opening
- 33: barrier sheet constricting means
- 34L: barrier sheet elastic member (barrier sheet constricting means)
- 34R: barrier sheet elastic member (barrier sheet constricting means)
- 38: middle portion elastic member (middle portion constricting means)
- 39: middle portion elastic member (middle portion constricting means)
- 42: elasticized sheet
- 44: lateral portion
- 45: lateral portion

### DESCRIPTION OF THE BEST MODE FOR WORKING OF THE INVENTION

### <Example 1 - First Embodiment>

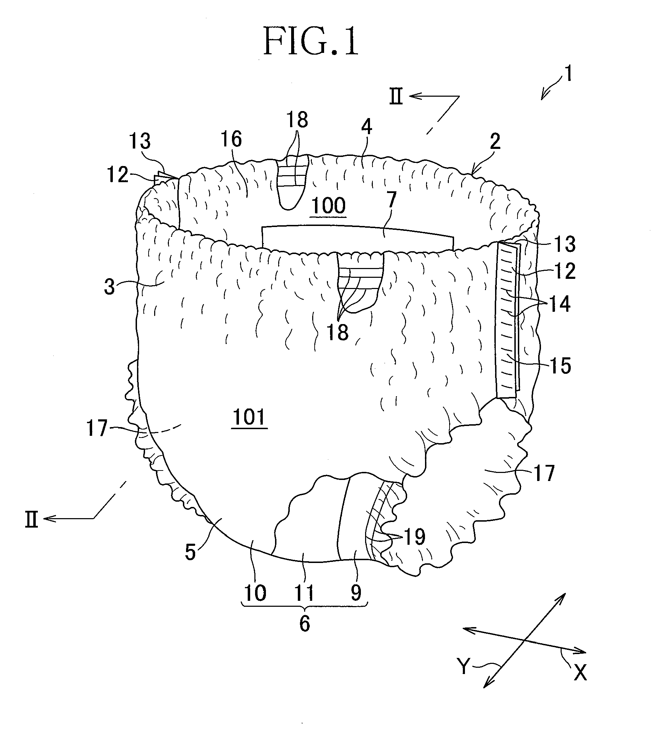

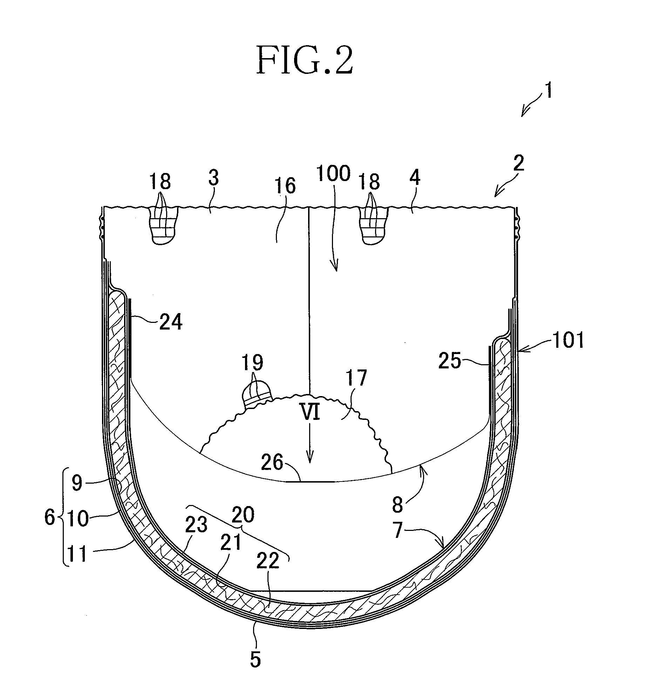

Figs. 1 through to 5 illustrate a first embodiment of the invention. Fig. 1 is a partially cutaway perspective view of a disposable diaper as a typical example of the absorbent article, and Fig. 2 is a sectional view taken along the line II-II of Fig. 1. Fig. 1 shows the diaper 1 in an arrangement suitable to be worn wherein the transverse direction and the longitudinal direction of the diaper 1 are indicated by arrows X and Y, respectively.

Referring to Fig. 1, the diaper 1 includes a liquid-absorbent chassis 2. The liquid-absorbent chassis 2 has an inner surface 100 facing the wearer's body, an outer surface 101 facing the wearer's garment, a front waist region 3, a rear waist region 4 and a crotch region 5 extending between these front and rear waist regions 3, 4. From a different viewpoint, the diaper 1 comprises a pant-shaped cover sheet 6, a liquid-absorbent structure 7 provided on the inside of the cover sheet 6, i.e., on the inner surface 100 facing the wearer' s body, and a barrier sheet 8 provided on the inside of the liquid-absorbent structure 7. The cover sheet 6 comprises, in turn, an inner sheet 9, an outer sheet 10 and a liquid- impervious sheet 11 sandwiched between these two sheets 9, 10, defining together the front waist region 3, the rear waist region 4 and the crotch region 5. The front and rear waist regions 3, 4 are put flat and joined together along respectively opposed waist side edges 12, 13 at a plurality of joins 14 arranged intermittently along these waist side edges 12, 13 to form seams 15.

The waist side edges 12, 13 are joined together at the joins 14 and thereby a waist-opening 16 is formed in a region surrounded by the front and rear waist regions 3, 4 and at the same time a pair of leg-openings 17 respectively surrounded by the joins 14 and the crotch region 5 are formed. A plurality of waist elastic members 18 extend along a peripheral edge of the waist-opening 16 and a plurality of leg elastic members 19 extend along peripheral edges of the respective leg-openings 17. These elastic members 18, 19 are sandwiched between the inner sheet 9 and the outer sheet 10 and bonded under tension to at least one of these sheets 9, 10 by means of adhesive (not shown).

Referring to Fig. 2, the liquid-absorbent structure 7 extends in the longitudinal direction Y between the front and rear waist regions 3, 4 but the liquid-absorbent structure 7 effectively functions so far as it is present at least in the crotch region 5. This liquid-absorbent structure 7 includes a liquid-absorbent panel 20 extending on the inner surface of the cover sheet 6.
The liquid-absorbent panel 20 comprises a liquid-absorbent core 22 wrapped with a liquid-absorbent and -spreadable sheet 21 such as tissue paper and an inner sheet 23 adapted to cover at least an area of the upper surface of the core 22 which faces the wearer's skin. The outer surface (bottom surface) of the panel 20 opposed to the inner sheet 23 is covered with the liquid-impervious sheet 11 by the intermediary of the outer sheet 10. The liquid-impervious sheet 11 may be sometimes directly bonded to the bottom surface of the liquid-absorbent panel 20.

In the front and rear waist regions 3, 4, at least a front end portion 24 and a rear end portion 25 of the barrier sheet 8 are bonded to the inner sheet 23 and, if it is necessary, along lateral portions 44, 45 also. The manner in which the barrier sheet 8 is attached is not limited to the illustrated embodiment and, for example, in the case of the diaper provided with the leak-barrier cuffs, it is also possible to bond the barrier sheet 8 to these leak-barrier cuffs.

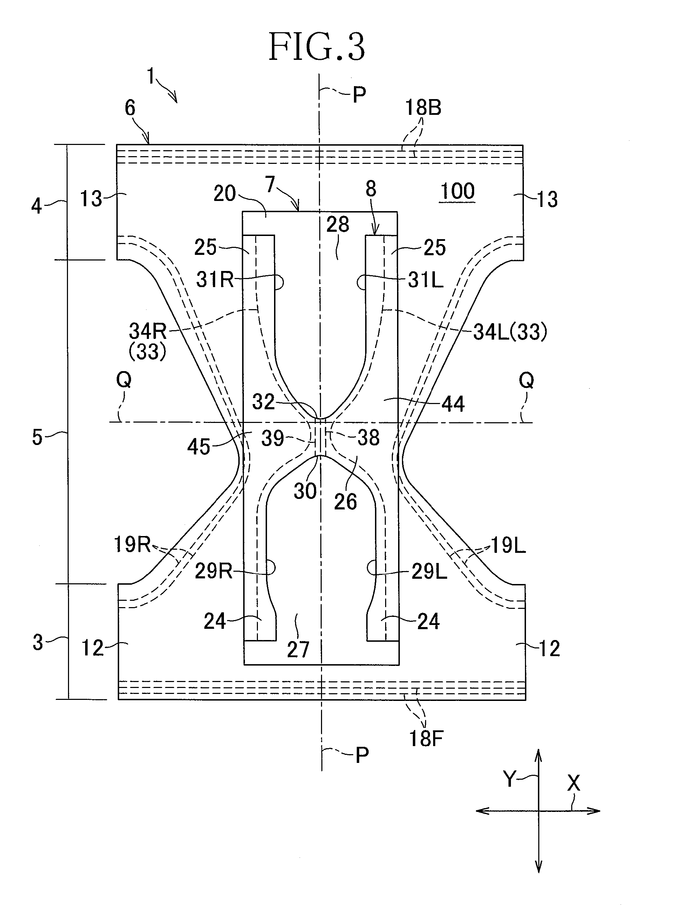

Fig. 3 is a plan view of the diaper 1 obtained by developing the diaper 1 in the longitudinal direction Y as well as in the transverse direction X after ripping the front and rear waist regions 3, 4 from each other along each column of the joins 14. In Fig. 3, the elastic members are held in a stretched state so that the diaper 1 may remain in a flat position. The expressions "left and right" used in the following description mean to be left and right relative to the wearer of the diaper. The diaper 1 has a longitudinal center line P-P bisecting a dimension of the diaper 1 in the transverse direction X and a transverse center line Q-Q bisecting a dimension of the diaper 1 in the longitudinal direction X. The diaper 1 is bilaterally-symmetric about the longitudinal center line P-P.

The cover sheet 6 is formed substantially in an hourglass-shape and the liquid-absorbent panel 20 is formed in a rectangular shape. The waist elastic members 18 provided on the cover sheet 6 comprise front and rear waist elastic members 18F, 18B as indicated by the broken lines, while the leg elastic members 19 comprise left and right leg-surrounding elastic members 19L, 19R are also indicated by broken lines.

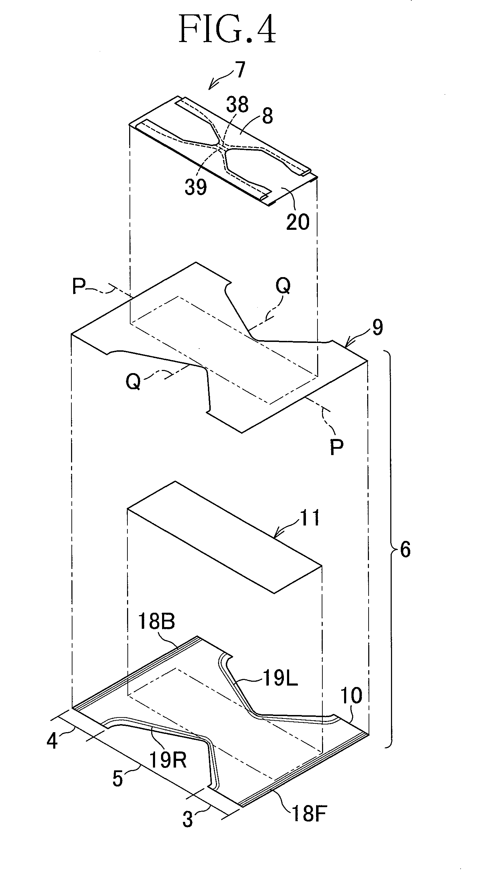

As will be apparent from Fig. 4 which is an exploded perspective view of the diaper 1, the liquid-absorbent panel 20 is laminated on the cover sheet 6. The outer sheet 10 constituting the cover sheet 6 is formed from a fibrous nonwoven fabric or moisture-pervious plastic film of which the inner surface (i.e., upper surface as viewed in Fig. 4) facing the wearer's skin is provided with the waist elastic members 18F, 18B and the leg elastic members 19L, 19R attached under tension thereto by means of hot melt adhesive (not shown). The liquid-impervious sheet 11 formed from a liquid-impervious and moisture-pervious plastic film is attached to the inner surface of the outer sheet 10 by means of adhesion or welding. As has previously been described, this liquid-impervious sheet 11 may be directly bonded to the bottom surface of the liquid-absorbent panel 20 and, in this case, the liquid-impervious sheet 11 preferably has at least an area sufficient to cover the bottom surface of the liquid-absorbent core 22 in order to achieve a liquid-impervious effect.

The liquid-impervious sheet 11 is provided on an inner surface with the inner sheet 9 formed from a fibrous nonwoven fabric or moisture-pervious plastic film and having a shape and size the same as those of the outer sheet 10 bonded thereto by means of adhesion or welding. The inner sheet 9 is provided, in turn, on its inner surface with the liquid-absorbent panel 20 bonded thereto. The liquid-absorbent panel 20 is intermittently coated on substantially the entirety of its outer surface (i.e., the bottom surface as viewed in Fig. 4) with hot melt adhesive (not shown) by means of which the liquid-absorbent panel 20 is bonded to the inner sheet 9. The liquid-absorbent panel 20 is provided on its inner surface with the barrier sheet 8.

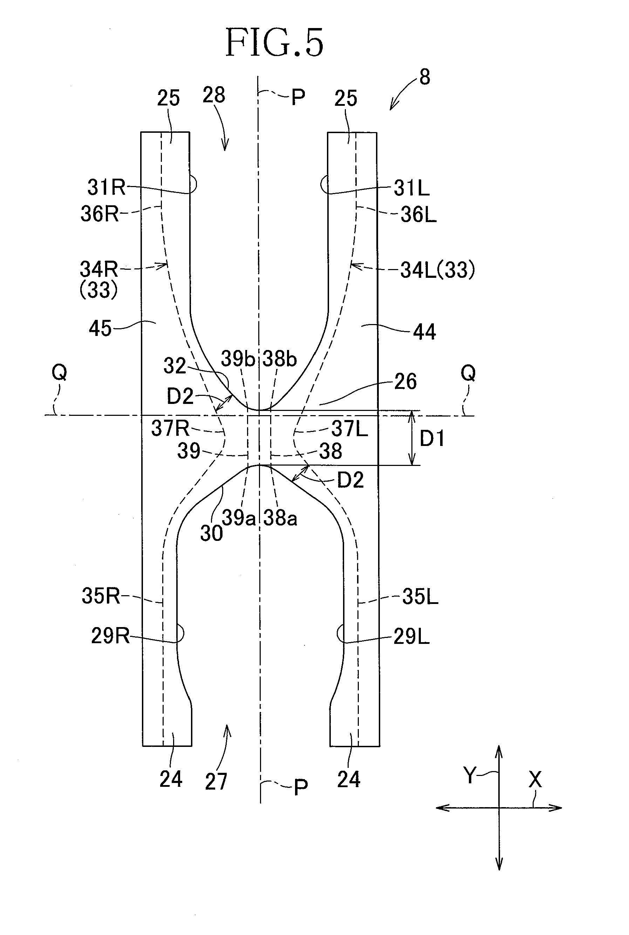

Fig. 5 illustrates the barrier sheet 8 of Figs. 3 and 4 in an enlarged scale. While the remaining members of the diaper 1 are not shown in Fig. 5, it should be assumed that the barrier sheet 8 is affixed to the diaper 1.
The barrier sheet 8 is formed from a sheet material such as fibrous nonwoven fabric, moisture-pervious plastic film or laminate thereof and is preferably liquid-impervious. The barrier sheet 8 has lateral portions 44, 45 opposed to and spaced from each other in the transverse direction X and a middle portion 26 extending between the lateral portions 44, 45 wherein the middle portion 26 is present in the crotch region 5. The lateral portions 44, 45 cooperate with the middle portion 26 to define passage openings, specifically, a substantially U-shaped front passage opening 27 extending from the middle portion 26 toward the front waist region 3 and a substantially U-shaped rear passage opening 28 extending from the middle potion 26 toward the rear waist region 4.

The front passage opening 27 is defined by two inner side edges 29L, 29R of the respective lateral portions 44, 45 and a curved closure edge 30 connecting the inner side edges 29L, 29R with each other, and the rear passage opening 28 is defined by two inner side edges 31L, 31R and a curved closure edge 32 connecting these inner side edges 31L, 31R with each other. The front passage opening 27 and the rear passage opening 28 respectively extend from the front and rear end portions 24, 25 of the barrier sheet 8 toward the middle portion 26 so that the region of the sheet allocated to come in direct contact with the wearer's skin may be as limited as possible and undesired stuffiness due to sweat or other secretions may be prevented. Although not illustrated, it is possible to form the barrier sheet 8 to have substantially the same size and shape as those of the inner sheet 9 defining a part of the cover sheet 6 and/or the front and rear passage openings 27, 28 are closed in the front and rear waist regions 3, 4.

The barrier sheet 8 is provided with barrier sheet constricting means 33 by which the barrier sheet 8 is spaced upward from the liquid-absorbent structure 7 in a thickness direction of the latter. For example, barrier sheet elastic members 34L, 34R each preferably comprising a single rubber thread may be used as such means 33, wherein the respective elastic members 34L, 34R extend in the longitudinal direction Y immediately along inner side edges 29L, 29R defining the front passage opening 27 and immediately along inner side edges 31L, 31R defining the rear passage opening 28. In the diaper 1 having its crotch region 5 concavely curved in the longitudinal direction Y as seen in Figs. 1 and 2, these elastic members 34L, 34R contract in the longitudinal direction Y to reduce the dimension of the barrier sheet 8 in the direction Y. Consequentially, a free region of the barrier sheet 8 inclusive of the middle portion 26 and opposed to the inner sheet 23 is spaced from the inner sheet 23 in the thickness direction of the diaper 1 (See Fig. 2).

When the diaper 1 is put on the wearer' s body, the relative position of the diaper 1 and the wearer' s body should be adjusted so that the front passage opening 27 is opposed to the wearer's external genitals, the rear passage opening 28 is opposed to the wearer's anus, and the middle portion 26 comes in contact with the wearer' s skin in a region defined between the external genital and the anus. With the diaper 1 put on the wearer's body in this manner, preferably the closure of the edge 30 of the front passage opening 27 is placed aside forward from the transverse center line Q-Q and the closure edge 32 of the rear passage opening 28 is just on, or in the vicinity of the transverse center line Q-Q.

The barrier sheet elastic member 34L comprises first and second segments 35L, 36L extending on opposite end sides as viewed in the longitudinal direction Y and a third segment 37L extending between these first and second segments 35L, 36L. The first and second segments 35L, 36L describe substantially straight paths extending along inner side edges 29L, 31L of the front and rear passage openings 27, 28, respectively, and the third segment 37L describes a path extending along the closure edges 30, 32 in the middle portion 26 so as to be convex toward the longitudinal center line P-P.

The barrier sheet elastic member 34R comprises first and second segments 35R, 36R extending on opposite end sides as viewed in the longitudinal direction Y and a third segment 37R extending between these first and second segments 35R, 36R. The first and second segments 35R, 36R describe substantially straight paths extending along inner side edges 29R, 31R of the front and rear passage openings 27, 28, respectively, and the third segment 37R describes a path extending along the closure edges 30, 32 in the middle portion 26 so as to be convex toward the longitudinal center line P-P. Along the third segments 37L, 37R, a dimension D2 between the barrier sheet elastic members 34L, 34R and the closure edges 30, 32, respectively, broaden as the barrier sheet elastic members 34L, 34R come close to the longitudinal center line P-P.

The middle portion 26 is provided with means for the middle portion 26. This means comprises middle portion elastic members 38, 39. These middle portion elastic members 38, 39 are provided between the third segments 37L, 37R of the barrier sheet elastic members 34L, 34R as viewed in the transverse direction X and extend in the longitudinal direction Y. Specifically, these middle portion elastic members 38, 39 have respective one ends 38a, 39a bonded under tension on the side of the closure edge 30 of the middle portion 26 and the respective other ends 38b, 39b fixed under tension on the side of the closure edge 32 of the middle portion 26.

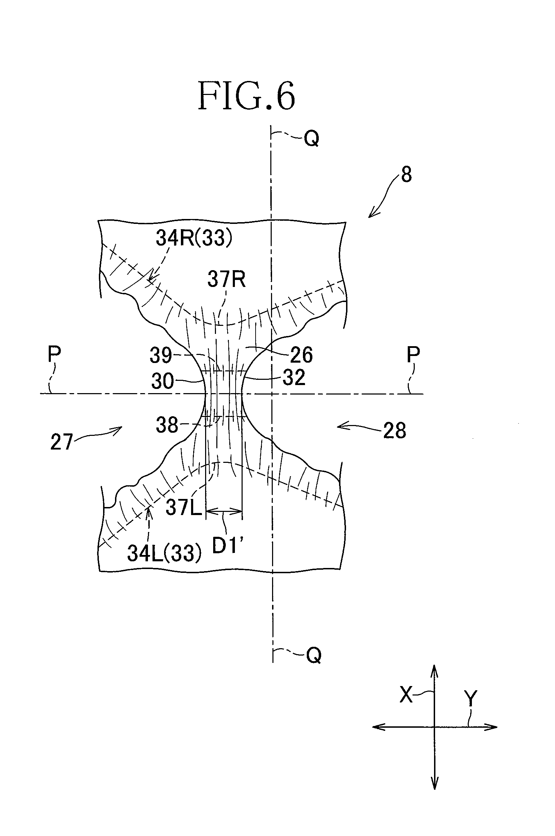

The middle portion elastic members 38, 39 bonded in the manner as has been described above cause the middle portion 26 to be constricted in the longitudinal direction Y under contractile force thereof as illustrated by Fig. 6 which is a scale-enlarged view of the middle portion 26 after contraction as viewed in a direction of the arrow VI in Fig. 2. As illustrated, the middle portion 26 is constricted under contraction of the middle portion elastic members 38, 39 in the longitudinal direction Y to a dimension D1'. Thus, the dimension D1' as measured after constriction becomes shorter than the dimension D1 (see Fig. 5) as measured before constriction. The reduced dimension of the middle portion 26 in the longitudinal direction Y allows an area of the middle portion 26 coming in contact with the wearer's skin to be correspondingly reduced. In consequence, the degree of irritation experienced by the wearer's skin can be correspondingly alleviated and uncomfortable feelings such as the feeling of discomfort otherwise experienced by the wearer can also be correspondingly alleviated.

A specific configuration of the middle portion 26 depends on those of the front and rear passage openings 27, 28 and the middle portion 26 is configured to be gradually broadened in the transverse direction X so as to be contiguous to the respective bottoms of the U-shaped front and rear passage openings as seen in Fig. 5. If the front and rear passage openings 27, 28 are configured to be rectangular, the middle portion 26 will also be configured to be rectangular. Placement of the middle portion elastic members 38, 39 may depend on the configurations of the middle portion 26. For the case of Fig. 5 in which the middle portion 26 is configured to be gradually broadened in the transverse direction so as to be contiguous to the bottom of the respective U-shaped front and rear passage-openings and the third segments 37L, 37R of the barrier sheet elastic members 34L, 34R are convexly curved toward the longitudinal center line P-P, the middle portion elastic members 38, 39 are preferably provided so as to extend in the vicinity of, and substantially in parallel to, the longitudinal center line P-P. In this way, the middle portion elastic members 38, 39 can minimize an affection of the contractile force of the third segments 37L, 37R.

The dimensions of the various components comprising the disposable diaper according to the present invention depend on whether the diaper is made exclusively for a baby or an adult and also on whether the diaper is of the pant-type or the open-type. In general, however, the dimension D1 (See Fig. 5) of the middle portion 26 as measured on the longitudinal center line P-P is preferably in a range of between around 20 to 40 mm and more preferably of 30 mm. The dimension in such a range are sufficient to prevent the middle portion 26 from being broken off in the course of forming the barrier sheet 8 with the passage openings.

While each of the middle portion elastic members 38, 39, when under tension, preferably has the same length as the dimension D1, each of these elastic members 38, 39 under tension may have a dimension longer than the dimension D1 at least by 30% or more.
Furthermore, each of the middle portion elastic members 38, 39 desirably has a stretch ratio higher than the stretch ratio of the barrier sheet elastic members 34L, 34R exhibited by the barrier sheet elastic members 34L, 34R in the middle portion 26. In the middle portion 26, the barrier sheet elastic members 34L, 34R are convexly curved inwardly and have respective biasing effects dispersed in the longitudinal direction Y and the transverse direction X. Therefore, the middle portion elastic members 38, 39 must overcome the biasing effect in the transverse direction X in order to constrict the middle portion 26 in the longitudinal direction Y. The stretch ratio may be set, for example, in a manner as will be described. First, the length of the respective middle portion elastic member 38, 39 before being stretched is measured. Then the elastic members 38, 39 are stretched to threefold their length and thus the stretch ratio of 3 is set. In such a stretched state, the middle portion elastic members 38, 39 are attached to the middle portion 26. For the barrier sheet elastic members 34L, 34R also, the length of the respective elastic members 34L, 34R before stretching is measured and then these elastic members 34L, 34R are stretched at a stretch ratio of 2.5. In such a stretched state, the elastic members 34L, 34R are attached to the associated side edges. It should be noted here that the barrier sheet elastic members are curved along the intermediate segments thereof and the length of the respective barrier sheet elastic members before and after stretching is measured along these intermediate segments. The stretch ratio of the elastic members 34L, 34R is set along these curved segments.

A distance between the middle portion elastic members 38, 39 is preferably in a range of about 10 to 40 mm. The distance between the middle portion elastic members 38, 39 depends on a specific configuration of the middle portion 26 as well as on a distance between the barrier sheet elastic members 34L, 34R, the desired function of the middle portion elastic members 38, 39 can be ensured so far as these elastic members 38, 39 are present between the barrier sheet elastic members 34L, 34R. However, the middle portion elastic members 38, 39 should be spaced from the associated barrier sheet elastic members 34L, 34R by a predetermined distance in order to alleviate a possibility that the elastic members 38, 39 might be affected by the contractile force of the elastic members 34L, 34R.

In this embodiment, a distance D2 by which the third segments 37L, 37R of the barrier sheet elastic members 34L, 34R are spaced from the associated closure edges 30, 32 is set to be gradually enlarged as these elastic members get close to the longitudinal center line P-P. Under contraction of the barrier sheet elastic members 34L, 34R, regions of the barrier sheet defined between the closure edges 30, 32 and the barrier sheet elastic members 34L, 34R, respectively, rise toward the wearer's skin. The regions of the barrier sheet rising up in this manner are relatively soft and comfortable in comparison to the remaining regions in which the barrier sheet elastic members 34L, 34R are attached to the barrier sheet. Therefore, the third segments 37L, 37R may be spaced from the associated closure edges 30, 32 as widely as possible to enlarge the uprising regions of the barrier sheet and thereby to alleviate a possible irritation of the wearer's skin.

While the middle portion 26 is provided with a pair of middle portion elastic members 38, 39 in this embodiment, it is possible, without departing from the scope of the invention, to provide the middle portion 26 with a single elastic member extending along the longitudinal center line P-P of this middle portion 26. In short, the elastic member for the middle portion 26 is not limited to any specific embodiment so far as the middle portion 26 can be constricted in the longitudinal direction Y from its initial dimension D1 as measured in a step of forming the front and rear passage openings 27, 28.
The barrier sheet elastic members 34L, 34R as the barrier sheet constricting means may be replaced by an elasticized sheet adapted to be stretchable and contractible at least in the longitudinal direction Y. In this case, the barrier sheet 8 as a whole may be elasticized so that such a barrier sheet 8 may be spaced upwardly from the liquid-absorbent structure in the thickness direction. Any other means may be adopted so far as the adopted means can function to space the barrier sheet 8 from the liquid-absorbent structure.

### <Example 2 - Second Embodiment>

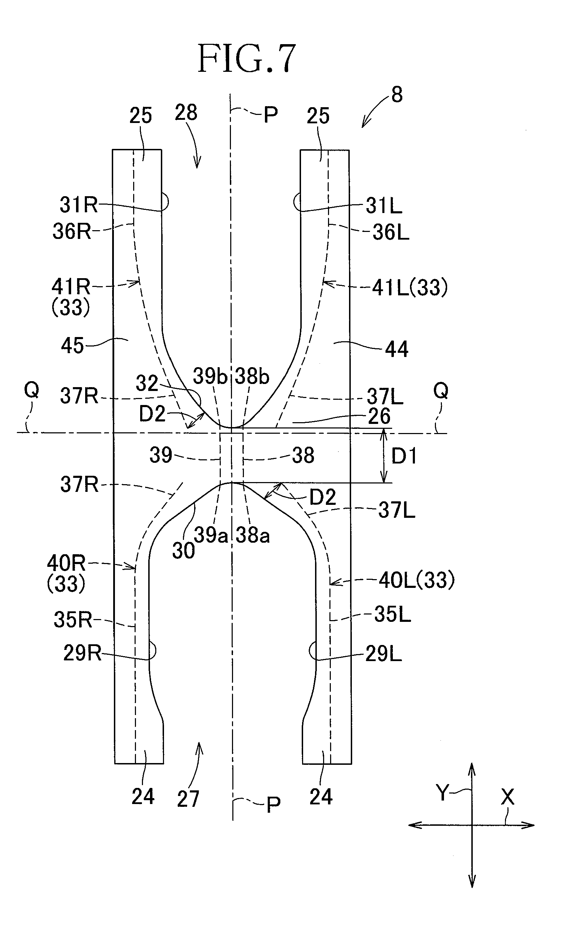

Fig. 7 is a view similar to Fig. 5 of the first embodiment, illustrating a second embodiment of the invention. This embodiment is **characterized in that** the barrier sheet elastic members serving as the barrier sheet constricting means 33 have the respective third segments 37L, 37R otherwise extending in the middle portion 26 cut off. The other constituent features are the same as those in the first embodiment and are designated by the same reference numerals as those used in the first embodiment and as such details thereof will not be described.

The barrier sheet elastic member provided on the left side of the wearer (corresponding to the right side with respect to the longitudinal center line P-P as viewed in Fig. 7) comprises a front elastic member 40L and a rear elastic member 41L. The front elastic member 40L comprises the first segment 35L and a front sub-segment of the third segment 37L while the rear elastic member 41L comprises the second segment 36L and a rear sub-segment of the third segment 37L. In this manner, the single barrier sheet elastic member has its third segment 37L cut off in the vicinity of the transverse center line Q-Q.

The barrier sheet elastic member provided on the right side of the wearer (corresponding to the left side with respect to the longitudinal center line P-P as viewed in Fig. 7) comprises a front elastic member 40R and a rear elastic member 41R. The front elastic member 40R comprises the first segment 35R and a front sub-segment of the third segment 37R, while the rear elastic member 41R comprises the second segment 36R and a rear sub-segment of the third segment 37R. In this manner, the single barrier sheet elastic member has its third segment 37R cut off in the vicinity of the transverse center line Q-Q.

The middle portion 26 is provided with the middle portion elastic members 38, 39 serving as the middle portion constricting means. These middle portion elastic members 38, 39 are present between the third segments 37L, 37R otherwise extending in the middle portion 26 as viewed in the transverse direction X and extend in the longitudinal direction Y substantially in parallel to the longitudinal center line P-P.

According to this second embodiment, none of the barrier sheet elastic members are present in the vicinity of the transverse center line Q-Q and no elastic force of the barrier sheet elastic members are exerted on the middle portion 26. Being free from the elastic force of the barrier sheet elastic members, it is possible to exert only the elastic force of the middle portion elastic members 38, 39 of the longitudinal direction Y on the middle portion 26 and thereby to constrict this middle portion 26 in the longitudinal direction Y. In consequence, it is possible to reduce an area of the middle portion 26 coming in contact with the wearer's skin and thereby to alleviate any irritation of the wearer's skin due to such contact.

### <Example 3 - Third Embodiment>

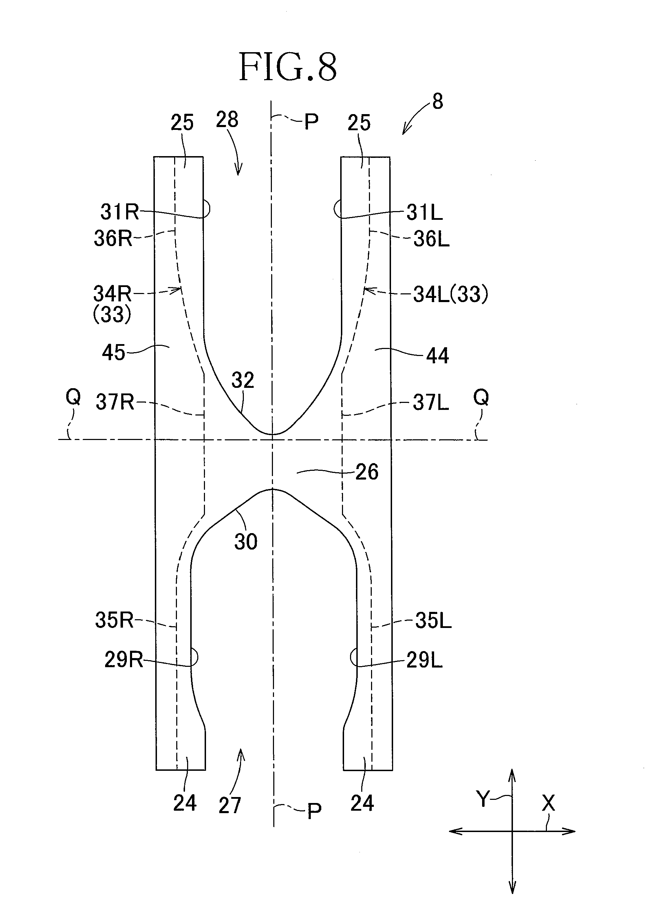

Fig. 8 is a view similar to Fig. 5 regarding the first embodiment, illustrating a third embodiment of the invention. This embodiment is **characterized in that** the barrier sheet elastic members 34L, 34R extend in the middle portion 26 substantially in parallel to the longitudinal center line P-P. The features common to those in the first embodiment are designated by the same reference numerals as those used in the first embodiment and details thereof will not be described.

The first segments 35L, 35R as well as the second segments 36L, 36R of the barrier sheet elastic members 34L, 34R are attached to the barrier sheet 8 so as to describe curves along the inner side edges 29L, 29R, 31L, 31R of the front and rear passage openings 27, 28 and, in the middle portion 26, the third segments 37L, 37R are attached to the barrier sheet 8 so as to extend in parallel to the longitudinal center line P-P. By attaching the barrier sheet elastic members 34L, 34R to the barrier sheet 8 in the middle portion so as to extend in parallel to each other, only the contractile force of the longitudinal direction Y is exerted on the middle portion 26. For example, if the barrier sheet elastic members are attached to the barrier sheet 8 so as to describe curves in the middle portion, the contractile force of these elastic members would be dispersed not only in the longitudinal direction Y but also in the transverse direction X. According to this embodiment, on the contrary, the middle portion 26 can be reliably constricted in the longitudinal direction Y without any fear of dispersing the contractile force in the transverse direction X.

According to this third embodiment, the contractile force exerted on the middle portion 26 can be limited to the longitudinal direction Y without additionally providing any separate member for use in constricting the middle portion 26.

## Claims

1. An absorbent article (1) comprising a liquid-absorbent chassis (2) having a longitudinal direction, a transverse direction, a side facing the article wearer's body (100), a side facing the wearer's garment (101), a front waist region (3), a rear waist region (4), a crotch region (5) extending between said front and rear waist regions (3, 4) and a liquid-absorbent structure (7) provided in said crotch region (5), and a barrier sheet (8) provided on said side facing the article wearer' s body of said liquid-absorbent chassis (2) and adapted to be spaced from said liquid-absorbent chassis (2) wherein said barrier sheet (8) includes barrier sheet constricting means (33) biasing said barrier sheet (8) to be constricted in said longitudinal direction and a space is formed between said barrier sheet (8) and said liquid-absorbent structure (7) as said liquid-absorbent structure (7) is bowed in said longitudinal direction, said absorbent article (1) being
**characterized in that:**
said barrier sheet (8) comprises a pair of lateral portions (44, 45) opposed to and spaced from each other in said transverse direction, a middle portion (26) connecting said lateral portions (44, 45) to each other, front and rear passage openings (27, 28) allowing passage of body waste into said space and, in addition to said barrier sheet constricting means (33), middle portion constricting means adapted to constrict said middle portion (26) in said longitudinal direction.

2. The absorbent article (1) as claimed in Claim 1, wherein said barrier sheet constricting means (33) comprises middle portion elastic members (38, 39) biasing said middle portion (26) to be constricted in the longitudinal direction and wherein said middle portion elastic members (38, 39) extend in parallel to a longitudinal center line bisecting a dimension of said middle portion (26) in said transverse direction and bonded under tension to said middle portion (26) so as to extend across said middle portion (26) in said longitudinal direction.

3. The absorbent article (1) as claimed in Claim 1 or Claim 2, wherein said barrier sheet constricting means (33) comprises barrier sheet elastic members (34L, 34R) extending in said longitudinal direction provided along respective inner edges defining said front and rear passage openings at least in said lateral portions.

4. The absorbent article (1) as claimed in Claim 3, wherein said barrier sheet elastic members (34L, 34R) are provided so as to be convexly curved, in said middle portion (26), toward a longitudinal center line bisecting the dimension of the barrier sheet (8) and to be gradually spaced from said inner edges as said barrier sheet elastic members (34L, 34R) become close to said longitudinal center line.

5. The absorbent article (1) as claimed in Claim 3 or Claim 4, wherein said barrier sheet constricting means (33) is set to a stretch ratio higher than that of said barrier sheet elastic members (34L, 34R).

6. An absorbent article (1) comprising a liquid-absorbent chassis (2) having a longitudinal direction, a transverse direction, a side facing the article wearer's body (100), a side facing the wearer's clothes (101), a front waist region (3), a rear waist region (4), a crotch region (5) extending between said front (3) and rear (4) waist region and a liquid-absorbent structure (7) provided in said crotch region (5), and a barrier sheet (8) provided on said side facing the article wearer' s body (100) of said liquid-absorbent chassis (2) and adapted to be spaced from said liquid-absorbent chassis (2) wherein said barrier sheet (8) includes barrier sheet constricting means (33) biasing said barrier sheet (8) to be constricted in said longitudinal direction such that a space is formed between said barrier sheet (8) and said liquid-absorbent structure as said liquid-absorbent structure is bowed in said longitudinal direction, said absorbent article being **characterized in that:**
said barrier sheet (8) comprises a pair of lateral portions (44, 45) opposed to and spaced from each other in said transverse direction, a middle portion (26) connecting said lateral portions to each other, and front and rear passage openings (27, 28) defined in front and rear zones of said crotch region by the intermediary of said middle portion (26) and allowing passage of body waste into said space; and
said barrier sheet constricting means (33) comprises barrier sheet elastic members (34L, 34R) extending in said longitudinal direction closely along respective inner edges defining said front and rear passage openings in said lateral portions and getting close to a longitudinal center line bisecting the dimension of the barrier sheet (8), extending in parallel to said longitudinal center line in said middle portion (26).
